# EUROPEAN PATENT SPECIFICATION

(11) **EP 2 102 220 B1**
(45) Date of publication and mention of the grant of the patent: **21.07.2010**
(21) Application number: 08701734.9
(22) Date of filing: 04.01.2008
(51) Int. Cl.: C07H 1/00, C07H 13/04

(54) **METHOD FOR THE SYNTHESIS OF SUCROSE-6-ESTERS**
VERFAHREN ZUR SYNTHESE VON SACCHAROSE-6-ESTERN
PROCÉDÉ DE SYNTHÈSE DE SUCROSE-6-ESTERS

(30) Priority: 09.01.2007 US 879501 P
(43) Date of publication of application: 23.09.2009
(73) Proprietor: Tate & Lyle Technology Limited, London EC3R 6DQ (GB)
(72) Inventor: BOUTZALE, Wayne, N., Saraland, AL 36571 (US); MICINSKI, Edward, Martinez, GA 30907 (US)
(74) Representative: Smyth, Gyles Darren
(86) International application number: PCT/GB2008/000013
(87) International publication number: WO 2008/084197

(56) References cited:
- EP-A- 0 475 619
- EP-A- 0 776 903
- WO-A-02/10180

## Description

This invention relates to an improved method for the synthesis of sucrose-6-esters.

Sucrose-6-esters are important intermediates in the synthesis of the artificial sweetener sucralose (4,1',6'-trichloro-4,1',6'-trideoxyg*alacto*sucrose).

A number of tin-mediated routes for the preparation of sucrose-6-esters have been disclosed, for example in US-4,950,746, US-5,023,329, and US-5,089,608. Each of these documents discloses a different organotin-based acylation promoter: US-4,950,746 employs a 1,3-di(hydrocarbyloxy)-1,1,3,3-tetra-(hydrocarbyl)distannoxane reagent; US-5,023,329 employs a di(hydrocarbyl)tin oxide; and US-5,089,608 employs the reaction product of a di(hydrocarbyl)tin oxide and a dihydric alcohol, an alkanolamine, or an enolizable α-hydroxyketone.

More particularly, EP-0 475 619-A discloses a process for producing a sucrose-6-ester, which comprises reacting sucrose with a carboxylic anhydride in a reaction mixture comprising a polar aprotic solvent and a 1,3-diacyloxy-1,1,3,3-tetra-(hydrocarbyl)distannoxane for a period of time sufficient to produce a sucrose-6-ester. In EP-0 475 619-A there is also disclosed a process for producing a sucrose-6-ester comprising slurrying sucrose and a 1,3-diacyloxy-1,1,3,3-tetra-(hydrocarbyl)distannoxane in a reaction mixture comprising a mixed solvent system containing a polar aprotic solvent and a hydrocarbon-like co-solvent capable of removing any water present in the reaction mixture by codistillation, and then, after removal of the water, treating the reaction mixture with a carboxylic acid anhydride.

An improvement in this method is disclosed in EP-0 776 903-A. That improvement comprises passing vapours of a solvent capable of removing water by codistillation (codistillation solvent) through a reaction mixture containing a polar aprotic solvent, sucrose, and a 1,3-diacyloxy-1,1,3,3-tetra-(hydrocarbyl)distannoxane, in sufficient quantity and sufficient time to remove substantially all the of the water in said reaction mixture by co-distillation.

An improvement in the method of EP-0 776 903-A is disclosed in WO 02/10180. WO 02/10180 discloses a process for the synthesis of a sucrose-6-ester comprising:
(a) reacting a mixture comprising sucrose and a polar aprotic solvent with an organotin-based acylation promoter, while adding a solvent capable of removing water by co-distillation, and removing water by co-distillation, to afford a first reaction mixture which is substantially free from water, followed by
(b) adding a carboxylic anhydride to said first reaction mixture to afford a second reaction mixture, and maintaining said second reaction mixture at a temperature and for a period of time sufficient to produce a sucrose-6-ester,
characterised in that
step (a) is performed at a temperature of from 85 to 125°C and at a pressure of from 20 to 80kPa.

In this prior art, the dehydration reaction to produce the sucrose-tin adduct is relatively slow. For example, in the Test Run of EP-0 776 903-A, the reaction is stated to be complete in 60 minutes (at atmospheric pressure). In WO 02/10180, the fastest reaction time disclosed is 18 minutes 57 seconds (at 33kPa). Longer reaction times are associated with the production of decomposed carbohydrates, which reduces the yield of sucrose-6-ester, hampers the extraction of the tin reagent at the end of the reaction, gives the product an undesired dark product.

Furthermore, in the prior art, significant amounts of unreacted sucrose remain in the product. For example, in the Test Run of EP-0 776 903-A, 1.65% sucrose remains, while in WO 02/10180 from 2.6% to 8.4% sucrose remains. As is disclosed in EP-0 776 903-A, residual sucrose is particularly undesirable, because it directly affects the yield of the sucrose-6-ester, and, after chlorination, forms undesirable chlorinated compounds (in particular tetrachlorosucroses) which are difficult to separate from the sucralose-6-ester and sucralose during subsequent processing.

According to the present invention, it has surprisingly been found that the dehydration reaction to produce the sucrose-tin adduct can be carried out faster, with consequential less production of decomposed carbohydrates, and with less unreacted sucrose, by conducting the reaction by contacting, in a continuous counter-current manner, a reaction mixture comprising sucrose, a polar aprotic solvent, and an organotin-based acylation promoter, with gas or solvent vapour capable of removing water.

It is an additional advantage of present invention that the dehydration reaction can be carried out in a truly continuous, rather than batch, fashion. Furthermore, surprisingly, it has been found that the process of the present invention gives very high selectivity of sucrose-6-ester over other esterified products.

According to the present invention, there is accordingly provided a process for the synthesis of a sucrose-6-ester comprising:
(a) forming a first reaction mixture comprising sucrose, a polar aprotic solvent, and an organotin-based acylation promoter;
(b) removal of water from said first reaction mixture by contacting, in a continuous counter-current manner, with gas or solvent vapour capable of removing water at a temperature, pressure and residence time sufficient to afford a second reaction mixture which is substantially free from water; followed by
(c) adding a carboxylic anhydride to said second reaction mixture to afford a third reaction mixture, and maintaining said third reaction mixture at a temperature and for a period of time sufficient to produce a sucrose-6-ester.

The present invention thus provides a process for forming the adduct between sucrose and the organotin-based acylation promoter by a reactive distillation.

The polar aprotic solvent is preferably *N,N*-dimethylformamide (DMF). Other suitable solvents are *N*-methyl-2-pyrrolidone, dimethyl sulfoxide, *N,N*-dimethylacetamide, and hexamethylphosphoramide. The choice is determined by the solubility of the sucrose and the 1,3-diacyloxy-1,1,3,3-tetra-(hydrocarbyl)distannoxane or other organotin-based acylation promoter (and the stannylated sucrose product) in the solvent, as well as by safety and toxicity considerations, especially if the sucrose-6-ester is to be used for the synthesis of sucralose, a food additive.

When the polar aprotic solvent is DMF, the DMF is preferably present in an amount of from 4 to 30g, preferably from 5 to 10g, more preferably from 6 to 8 g, per g of sucrose.

In the prior art, a solvent capable of removing water by codistillation, or vapours thereof, were always used to achieve the removal of water in the step corresponding to step (b). However, surprisingly, in the present invention it has been found that a gas can also be used. If a gas is used, the gas can be, for example, nitrogen, argon, air, helium, carbon dioxide, and is preferably nitrogen or argon. If solvent vapour is used, then any solvent whose vapours are able to remove water can be used. Preferred solvents are those which are immiscible with water and form a constant-composition minimum-boiling azeotrope with water. Exemplary classes of solvents are saturated hydrocarbons, aromatic hydrocarbons, chlorinated hydrocarbons, ketones, esters, and ethers. Specific examples are cyclohexane, *n*-heptane, isooctane (2,2,4-trimethylpentane), benzene, toluene, diethyl ether, chloroform, carbon tetrachloride, - hexane, ethyl acetate, and methyl acetate. In the same manner as in the selection of the polar aprotic solvent, safety and toxicity considerations will also affect the choice of an appropriate solvent. Hydrocarbons are preferred, and cyclohexane, *n-*heptane, toluene, and isooctane (2,2,4-trimethylpentane) are particularly preferred. The most preferred solvent is cyclohexane.

A wide variety of means can be used to achieve the continuous counter-current contacting in step (b), and these will be apparent to those skilled in the art. In principle, any counter-current mass-contacting device that promotes gas/liquid thermodynamic equilibrium can be employed. Conveniently, a column can be employed. The column can be a packed column, a tray column, or a packed tray column (that is, a column equipped with both trays and with packing). The number of trays to be employed in a tray column will be apparent to the person skilled in the art, having regard to the scale of the reaction, and the reaction conditions employed. Similarly, the packing possibilities for a packed column will likewise be apparent. Preferably, the column is a distillation column equipped with sieve trays with downcomers.

When a column is employed, the first reaction mixture will be fed at the top of the column, and the gas or solvent vapour will be fed at the bottom of the column. The second reaction mixture will emerge at the bottom of the column, and at the top of the column will emerge a stream consisting of the polar aprotic solvent, water, and the gas or solvent capable of removing water. From this stream the water can be easily separated and the gas and/or solvent(s) may be recycled.

The feed rate of the first reaction mixture and the feed rate of the gas or solvent vapour will vary according to the design of the device used for the continuous counter-current operation, and on the nature of the gas or solvent vapour employed, and these will easily be determined by those skilled in the art. When cyclohexane is used to remove water, the weight ratio of cyclohexane feed to sucrose feed is typically from 5 to 50, more preferably from 10 to 25, most preferably from 15 to 20. When nitrogen is used to remove water at atmospheric pressure, the weight ratio of nitrogen feed to sucrose feed is typically from 5 to 50, more preferably from 10 to 25, most preferably from 15 to 20.

It can be advantageous to mix a small amount of the polar aprotic solvent with the water-removing solvent. Typically, the amount of the polar aprotic solvent added will be from 1 to 20%, more preferably from 5 to 15% by weight.

The temperature of the dehydration reaction is generally to be maintained in the region of from 60 to 140°C, preferably from 85 to 125°C, more preferably from 90 to 110°C. One way to achieve this is to pre-heat both the first reaction mixture and the gas or solvent vapour. The first reaction mixture can be preheated to from 50°C above to 50°C below the highest temperature in the column or other device, preferably to from 20°C above to 30°C below the highest temperature in the column or other device. The gas or solvent vapour can be preheated to from 1 to 50°C above the highest temperature in the column or other device, preferably to from 10 to 30°C above the highest temperature in the column or other device.

The dehydration reaction is most conveniently carried out at atmospheric pressure. However, it is possible to vary the pressure, although this may vary the optimum temperature and residence time. The pressure can, for example, be from 2kPa to 400kPa. Conveniently, the pressure can be at or slightly above atmospheric pressure, for example from 101 to 110kPa.

The residence time will vary according to the temperature and pressure employed, and also on the scale of the reaction, and can be determined by the person skilled in the art. However, typical residence times on a laboratory scale are in the order of minutes, for example from 1 to 10 minutes. It is a totally unexpected feature of the present invention that the dehydration reaction, even at atmospheric pressure, could be achieved so quickly.

In general the present invention will be practised in a continuous manner. However, "continuous" as used herein does not preclude pausing the operation from time to time.

The formation of the first reaction mixture can be performed in a number of ways. A single stream comprising sucrose, the polar aprotic solvent, and the organotin-based acylation promoter can be formed. Alternatively two streams, one comprising sucrose and the other comprising the organotin-based acylation promoter can be formed, and these two streams can be mixed. In this case, the streams can be mixed before entering the column or other device, or they can be fed into the column or other device at separate points so that they come into contact inside the column or other device.

The organotin-based acylation promoter can be any of those known in the art *per se,* for example any of those disclosed in US-4,950,746, US-5,023,329, US-5,089,608, or EP-0 475 619-A. In particular, the organotin-based acylation promoter can be selected from the group consisting of: a 1,3-di(hydrocarbyloxy)-1,1,3,3-tetra-(hydrocarbyl)distannoxane; a di(hydrocarbyl)tin oxide; the reaction product of a di(hydrocarbyl)tin oxide and a dihydric alcohol, an alkanolamine, or an enolizable α-hydroxyketone; and a 1,3-diacyloxy-1,1,3,3-tetra-(hydrocarbyl)distannoxane. The organotin-based acylation promoter is preferably a 1,3-diacyloxy-1,1,3,3-tetra-(hydrocarbyl)distannoxane.

By "hydrocarbyl" as used herein, is meant an alkyl, cycloalkyl, aryl, or aralkyl group.

When the organotin-based acylation promoter is a 1,3-di(hydrocarbyloxy)-1,1,3,3-tetra-(hydrocarbyl)distannoxane, the hydrocarbyloxy group is preferably a C₁-C₈ alkoxy group or phenoxy, more preferably methoxy, ethoxy, n-propyloxy, n-butyloxy, n-pentyloxy or n-hexyloxy, and most preferably a methoxy group. The hydrocarbyl group in turn is preferably an alkyl group, more preferably a C₁-C₈ alkyl group, and most preferably an n-butyl group.

When the organotin-based acylation promoter is a di(hydrocarbyl)tin oxide, the hydrocarbyl group is preferably an alkyl group, more preferably a C₁-C₈ alkyl group, and most preferably an n-butyl group.

When the organotin-based acylation promoter is the reaction product of a di(hydrocarbyl)tin oxide and a dihydric alcohol, an alkanolamine, or an enolizable α-hydroxyketone, the di(hydrocarbyl)tin oxide is preferably as described above. The dihydric alcohol can be an alkane diol, preferably having from 2 to 8 carbon atoms. Suitable examples are ethylene glycol, 2,3-propanediol, 2,3-butanediol, 1,3-butanediol, 1,4-butanediol, 1,3-propanediol, 1,2-pentanediol, and 1,2-hexanediol. Alternatively, the dihydric alcohol can be a cycloalkane diol, preferably having from 5 to 8 carbon atoms. Suitable examples are 1,2-cyclohexanediol and 1,2-cyclopentanediol. In both cases, it is preferred that the two hydroxyl groups are not more than four carbon atoms distant from each other on the carbon chain to which they are bonded, and it is more preferred that they be on adjacent carbon atoms or that there be only one carbon atom separating the carbon atoms to which the hydroxyl groups are bonded. The alkanolamine is preferably a C₂-C₈ alkanolamine, and preferably the hydroxyl group and the amino group are not more than four carbon atoms distant from each other on the carbon chain to which they are bonded, and more preferably the hydroxyl group and the amino group are on adjacent carbon atoms or there is only one carbon atom separating the carbon atoms to which the hydroxyl group and the amino group are bonded. Suitable alkanolamines are ethanolamine, 2-amino-1-propanol, and 1-amino-2-propanol. Suitable enolisable α-hydroxyketones are benzoin (2-hydroxy-2-phenylacetophenone) and acetoin (3-hydroxy-2-butanone).

Preferably, however, the organotin-based acylation promoter is a 1,3-diacyloxy-1,1,3,3-tetra-(hydrocarbyl)distannoxane. The hydrocarbyl group of the 1,3-diacyloxy-1,1,3,3-tetra-(hydrocarbyl)distannoxane is preferably an alkyl group, more preferably a C₁-C₈ alkyl group, and most preferably a butyl group, so that 1,1,3,3-tetrabutyldistannoxanes are particularly preferred. It is convenient if the acyloxy group matches that of the carboxylic anhydride to be used, so that, for example, when a sucrose-6-acetate is being synthesised, 1,3-diacetoxy-1,1,3,3-tetrabutyldistannoxane (distannoxane diacetate DSDA) is most preferred.

When the organotin-based acylation promoter is a dinuclear species containing two atoms of tin per molecule (*e.g*. a distannoxane), it is preferably present in from 0.5 to 2.5 molar equivalents (per mole of sucrose), more preferably from 0.75 to 1.2 molar equivalents, still more preferably from 0.9 to 1.1 molar equivalents, and most preferably 1.0 molar equivalents.

When the organotin-based acylation promoter is a mononuclear species containing one atom of tin per molecule (*e.g*. a di(hydrocarbyl)tin oxide), it is preferably present in from 0.5 to 2.5 molar equivalents (per mole of sucrose), more preferably from 0.8 to 1.5 molar equivalents, and most preferably 1.2 molar equivalents.

When the organotin-based acylation promoter is a 1,3-diacyloxy-1,1,3,3-tetra-(hydrocarbyl)distannoxane, it is preferably recovered after the end of step (c) and reused. This can be achieved by partitioning the product mixture between water and cyclohexane, creating an upper 1,3-diacyloxy-1,1,3,3-tetra-(hydrocarbyl)distannoxane/cyclohexane phase, and a lower sucrose ester/DMF/water/acetic acid phase, and then further extracting the lower phase with cyclohexane. The combined cyclohexane extracts are then combined and concentrated, preferably under reduced pressure. The 1,3-diacyloxy-1,1,3,3-tetra-(hydrocarbyl)distannoxane can be further purified by conventional techniques. This is disclosed, for example, in US 5,034,551.

The removal of water in step (b) can be conveniently monitored by the weight ratio of tin to water in the reaction mixture. The tin content can measured using an X-Ray Fluoresence Analyzer, and the water content can be measured by the Karl-Fischer method. It has been found that the weight ratio of tin to water at the end of step (b) should be from 20 to 35, more preferably from 24 to 32, and most preferably from 26 to 28, for optimum yields.

The method of the present invention can be used to prepare a variety of sucrose-6-esters, provided that the appropriate carboxylic anhydride is available. In particular, the method can be used to prepare sucrose-6-acetate, by the use of acetic anhydride, and sucrose-6-benzoate, by the use of benzoic anhydride. As mentioned before, it is convenient, but not necessary, to then use an acyloxytin reagent having the same acyloxy group as the ester to be prepared.

The carboxylic anhydride is preferably added in an amount of from 0.8 to 1.5 molar equivalents (per mole of sucrose starting material), more preferably from 1.05 to 1.35 molar equivalents, still more preferably from 1.1 to 1.25 molar equivalents, and most preferably 1.15 molar equivalents. Too much carboxylic anhydride results in the formation of excessive amounts of dicarboxylate by-products, while too little results in large amounts of sucrose being recovered at the end of the reaction.

The esterification reaction can be conducted at from -10 to 60°C, preferably at from 0 to 10°C.

The sucrose-6-ester of the present invention is useful as an intermediate in the preparation of sucralose. The further steps to afford sucralose are: chlorination of the 4, 1', 6'-positions, deacylation, and isolation of the sucralose.

Thus, according to the present invention, there is also provided a process for the synthesis of sucralose comprising preparing a sucrose-6-ester by a process according to any of claims 1 to 19, chlorinating the 4, 1', 6' positions to produce a sucralose-6-ester, deacylation of the sucralose-6-ester, and isolation of the sucralose.

The chlorination can be achieved by methods known to those skilled in the art, for example by the methods of US 4,980,463.

The deacylation can be achieved by methods known to those skilled in the art, for example by the methods of US 4,980,463, US 5,530,106, US 5,498, 709, and US 6,890, 581.

### Brief Description of the Drawings

Figure 1 shows a schematic view of a process according to an embodiment of the present invention
Figure 2 shows a schematic view of an Oldershaw column used as the counter-current column in an embodiment of the present invention.

### Example

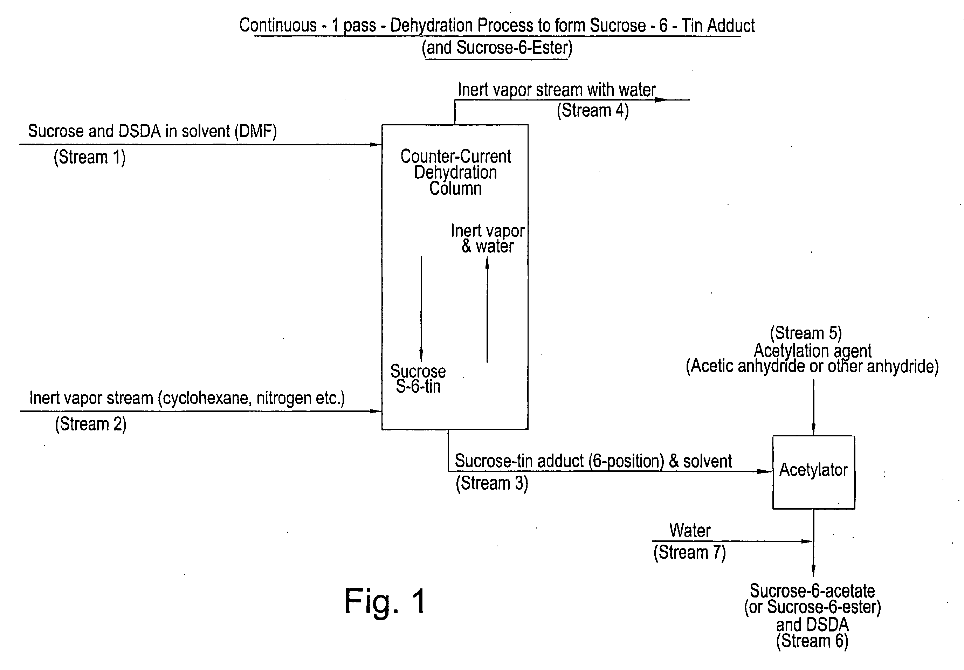

The experimental set-up is shown schematically in Figure 1.

As shown in Figure 1, sucrose dissolved in DMF or other suitable solvent is added at or near the top of the column (Stream 1). Either premixed with the sucrose, or added as a separate solution, dissolved DSDA or other tin species is also added at or near the top of the stripping column (Stream 1).

To the bottom of the column is added a suitable stripping solvent or inert gas (Stream 2). The column is maintained at a temperature and pressure such that the vapour pressure of the water allows the water of reaction to be transferred to the vapour phase. The vapour stream is maintained at sufficient temperature, pressure and flow as to efficiently remove the water as vapour from the vicinity of the sucrose/tin mixture. The water vapour and inert gas/vapour are removed from the top of the stripping column (Stream 4).

The carbohydrate stream containing the sucrose-6-tin adduct is removed from the bottom of the stripping column as a solution (Stream 3).

The solution of the sucrose-6-tin adduct is mixed with a suitable esterification agent such as acetic anhydride (Stream 5). After sufficient time at a temperature near or below ambient temperature, the mixture is quenched with water (Stream 7) and the resulting sucrose-6-ester is obtained (Stream 6). The DSDA or other tin species also obtained in Stream 6 is available for recycle.

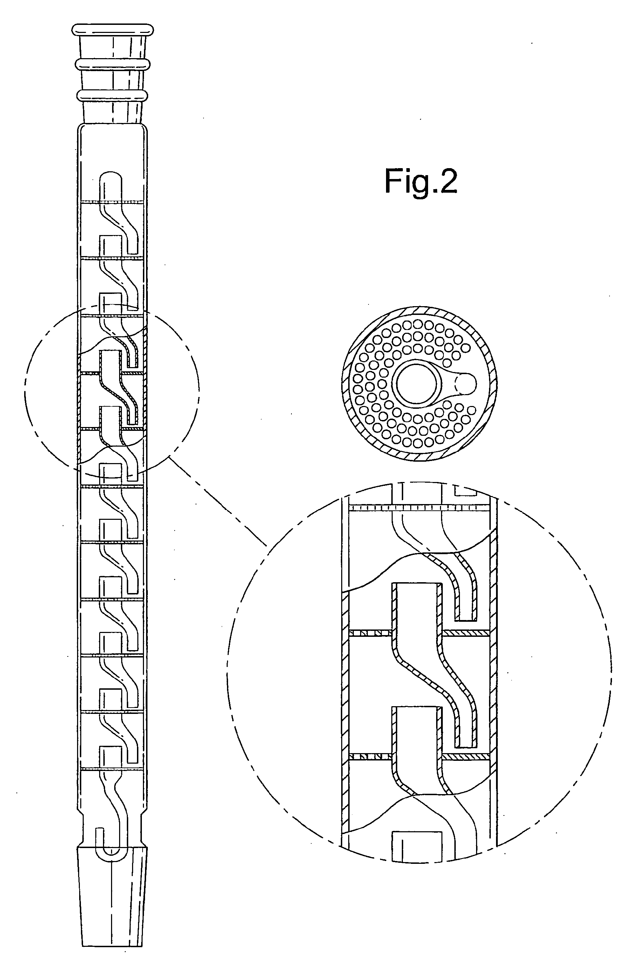

Procedure:
1. A 1 litre flask was charged with 30.08 grams of sugar and 200.05 grams of DMF. The mixture was stirred and heated to 90°C to dissolve the sugar. After cooling, 80.20 grams of DSDA (66.9%) in cyclohexane) was added with stirring.
2. The mixture in step 1 was fed continuously to the top of a 20 plate Oldershaw column (shown schematically in Figure 2) at a rate of 6ml/min (6.24g/min). Simultaneously, cyclohexane vapor was fed to the bottom of the column at a rate of 9.2g/min. Vapors containing cyclohexane, water and DMF from the overheads of the column were condensed and collected without allowing the condensate to return to the column. After operation of the column for 17 minutes, the process was at steady state and collection of a sample of the liquid from the bottom of the column was started. The sample from the bottom of the column was collected for a 20 minute period. This product was a clear, light amber color. Column residence time (reaction time) was calculated to be 4.8 minutes.
3. The sample from the bottom of the column was calculated to contain an equivalent of 12.1 grams of sugar. (6.24g/min X 20 min X 9.69% sugar = 12.1 grams of sugar). This sample was acetylated with 4.31 grams of acetic anhydride at <10°C. After 2 hours at <10°C, the sample was quenched with 5.1 I grams of water. The weight of the sample at this point was 107 grams. The material was extracted with 50ml of cyclohexane to remove the tin catalyst.
4. The product from step 3 was analyzed by HPLC as follows:

| | |
|---|---|
| a. Sucrose-6-Acetate = | 6.15% (84.9% normalized) |
| b. Diacetates = | 1.06% (14.6% normalized) |
| c. Monoacetates = | 0.01% (0.14% normalized) |
| d. Sucrose = | 0.02% (0.28% normalized) |

## Claims

1. A process for the synthesis of a sucrose-6-ester comprising:
(a) forming a first reaction mixture comprising sucrose, a polar aprotic solvent, and an organotin-based acylation promoter;
(b) removal of water from said first reaction mixture by contacting, in a continuous counter-current manner, with gas or solvent vapour capable of removing water at a temperature, pressure and residence time sufficient to afford a second reaction mixture which is substantially free from water; followed by
(c) adding a carboxylic anhydride to said second reaction mixture to afford a third reaction mixture, and maintaining said third reaction mixture at a temperature and for a period of time sufficient to produce a sucrose-6-ester.

2. A process according to Claim 1 wherein said polar aprotic solvent is dimethylformamide.

3. A process according to Claim 1 or Claim 2 wherein said solvent capable of removing water is cyclohexane.

4. A process according to Claim 1 or Claim 2 wherein said gas capable of removing water is nitrogen.

5. A process according to any of Claims 1 to 4 wherein a counter-current mass-contacting device that promotes gas/liquid thermodynamic equilibrium is employed to achieve the continuous counter-current contacting in step (b).

6. A process according to Claim 5 wherein the device is a column.

7. A process according to Claim 6 wherein the column is a packed column, a tray column, or a packed tray column.

8. A process according to Claim 7 wherein the column is a distillation column equipped with sieve trays and downcomers.

9. A process according to any of Claims 1 to 8, wherein said organotin-based acylation promoter is a 1,3-diacyloxy-1,1,3,3-tetra-(hydrocarbyl)distannoxane.

10. A process according to Claim 9, wherein said 1,3-diacyloxy-1,1,3,3-tetra-(hydrocarbyl)distannoxane is 1,3-diacyloxy-1,1,3,3-tetrabutyldistannoxane.

11. A process according to Claim 9 or Claim 10, wherein the 1,3-diacyloxy-1,1,3,3-tetra-(hydrocarbyl)distannoxane is recovered and re-used.

12. A process according to any of Claims 1 to 11, wherein said sucrose-6-ester is a sucrose-6-acetate, and said carboxylic anhydride is acetic anhydride.

13. A process for the synthesis of sucralose comprising preparing a sucrose-6-ester by a process according to any of claims 1 to 12, chlorinating the 4, 1', 6' positions to produce a sucralose-6-ester, deacylation of the sucralose-6-ester, and isolation of the sucralose.

## Patentansprüche

1. Verfahren zur Synthese eines Saccharose-6-Esters, umfassend:
(a) Bilden einer ersten Reaktionsmischung umfassend Saccharose, ein polares aprotisches Lösungsmittel und einen Organozinnbasierten Acylierungspromotor;
(b) Entfernen von Wasser aus der Reaktionsmischung durch Kontaktierung, in einem kontinuierlichen Gegenstrom, mit Gas oder Dampf eines Lösungsmittels, die in der Lage sind Wasser bei einer Temperatur, einem Druck und einer Verweilzeit zu entfernen, die ausreichend sind, um eine zweite Reaktionsmischung hervorzubringen, die im Wesentlichen wasserfrei ist; gefolgt von
(c) Hinzufügen eines Carbonsäureanhydrids zur zweiten Reaktionsmischung, um eine dritte Reaktionsmischung hervorzubringen, und Halten der dritten Reaktionsmischung bei einer Temperatur und für eine Zeitdauer, die ausreichen um Saccharose-6-Ester herzustellen.

2. Verfahren nach Anspruch 1, wobei das polare aprotische Lösungsmittel Dimethylformamid ist.

3. Verfahren nach Anspruch 1 oder Anspruch 2, wobei das Lösungsmittel, das in der Lage ist Wasser zu entfernen, Cyclohexan ist.

4. Verfahren nach Anspruch 1 oder Anspruch 2, wobei das Gas, das in der Lage ist Wasser zu entfernen, Stickstoff ist.

5. Verfahren nach einem der Ansprüche 1 bis 4, wobei eine Gegenstrom-Masse-Kontaktierungsvorrichtung, die ein thermodynamisches Gleichgewicht von Gas/Flüssigkeit fördert, angewendet wird, um die kontinuierliche Gegenstrom-Kontaktierung in Schritt (b) zu erhalten.

6. Verfahren nach Anspruch 5, wobei die Vorrichtung eine Kolonne ist.

7. Verfahren nach Anspruch 6, wobei die Kolonne eine gepackte Säule, eine Bodenkolonne oder eine gepackte Bodenkolonne ist.

8. Verfahren nach Anspruch 7, wobei die Kolonne eine Destillationskolonne ist, die mit Siebböden und Ableitern versehen ist.

9. Verfahren nach einem der Ansprüche 1 bis 8, wobei der Organozinnbasierte Acylierungspromotor 1,3-Diacyloxy-1,1,3,3-tetra(hydrocarbyl)-distannoxan ist.

10. Verfahren nach Anspruch 9, wobei das 1,3-Diacyloxy-1,1,3,3-tetra(hydrocarbyl)-distannoxan 1,3-Diacyloxy-1,1,3,3-tetrabutyldistannoxan ist.

11. Verfahren nach Anspruch 9 oder Anspruch 10, wobei das 1,3-Diacyloxy-1,1,3,3-tetra(hydrocarbyl)-distannoxan rückgewonnen und wiederverwendet wird.

12. Verfahren nach einem der Ansprüche 1 bis 11, wobei der Saccharose-6-Ester ein Saccharose-6-Azetat ist, und das Carbonsäureanhydrid Essigsäureanhydrid ist.

13. Verfahren zur Synthese von Sucralose umfassend das Bereitstellen eines Saccharose-6-Esters durch ein Verfahren nach einem der Ansprüche 1 bis 12, Chlorieren der 4,1',6'-Positionen zur Herstellung eines Saccharose-6-Esters, Deacylierung des Saccharose-6-Esters und Isolierung der Sucralose.

## Revendications

1. Procédé de synthèse de 6-ester de saccharose comprenant:
(a) la formation d'un premier mélange réactionnel comprenant du saccharose, un solvant aprotique polaire, et un promoteur d'acylation à base d'organoétain;
(b) l'élimination de l'eau dudit mélange réactionnel par la mise en contact, à contre-courant continu, avec un gaz ou une vapeur de solvant capable d'éliminer l'eau à une température, une pression et un temps de séjour suffisants pour donner un deuxième mélange réactionnel qui est substantiellement exempt d'eau; suivi par
(c) l'ajout d'un anhydride carboxylique audit deuxième mélange réactionnel pour donner un troisième mélange réactionnel, et maintien dudit troisième mélange réactionnel à une température et durant une période de temps suffisants pour produire un 6-ester de saccharose.

2. Procédé selon la revendication 1, dans lequel ledit solvant polaire aprotique est le diméthylformamide.

3. Procédé selon la revendication 1 ou 2, dans lequel ledit solvant capable d'éliminer l'eau est le cyclohexane.

4. Procédé selon la revendication 1 ou 2, dans lequel ledit gaz capable d'éliminer l'eau est l'azote.

5. Procédé selon les revendications 1 à 4, dans lequel un dispositif de contact de masse à contre-courant favorisant un équilibre thermodynamique gaz/liquide est employé pour obtenir le contact à contre courant continu de l'étape (b).

6. Procédé selon la revendication 5, dans lequel le dispositif est une colonne.

7. Procédé selon la revendication 6, dans lequel la colonne est une colonne garnie, une colonne à plateau ou une colonne garnie à plateau.

8. Procédé selon la revendication 7, dans lequel la colonne est une colonne de distillation équipée de plateaux perforés et de déversoirs.

9. Procédé selon l'une quelconque des revendications 1 à 8, dans lequel ledit promoteur d'acylation à base d'organoétain est un 1,3-diacyloxy-1,1,3,3-tétra(hydrocarbyl)distannoxane.

10. Procédé selon la revendication 9, dans lequel ledit 1,3-diacyloxy-1,1,3,3-tétra(hydrocarbyl)distannoxane est le 1,3-diacyloxy-1,1,3,3-tétrabutyldistannoxane.

11. Procédé selon la revendication 9 ou la revendication 10, dans lequel le 1,3-diacyloxy-1,1,3,3-tétra-(hydrocarbyl)distannoxane est récupéré et réutilisé.

12. Procédé selon l'une quelconque des revendications 1 à 11, dans lequel ledit 6-ester de saccharose est un 6-acétate de saccharose, et ledit anhydride carboxylique est un anhydride acétique.

13. Procédé de synthèse de sucralose comprenant la préparation d'un 6-ester de saccharose par un processus selon l'une quelconque des revendications 1 à 12, la chloration des positions 4, 1', 6' pour produire un 6-ester de sucralose, la déacylation du 6-ester de sucralose, et l'isolation du sucralose.
